# EUROPEAN PATENT SPECIFICATION

(11) **EP 3 539 519 B1**
(45) Date of publication and mention of the grant of the patent: **09.12.2020**
(21) Application number: 19156834.4
(22) Date of filing: 13.02.2019
(51) Int. Cl.: A61F 13/551, A61F 13/496

(54) **FOLDED ABSORBENT ARTICLES**
GEFALTETE SAUGFÄHIGE ARTIKEL
ARTICLES ABSORBANTS PLIÉS

(30) Priority: 16.03.2018 EP 18162245
(43) Date of publication of application: 18.09.2019
(73) Proprietor: The Procter & Gamble Company, Cincinnati, OH 45202 (US)
(72) Inventor: YAMAMOTO, Yoichiro, 53881 Euskirchen (DE)
(74) Representative: P&G Patent Germany

(56) References cited:
- JP-A- H1 095 481
- US-A1- 2006 218 700
- US-A1- 2008 134 641
- US-A1- 2012 043 244
- US-A1- 2013 130 879

## Description

### FIELD OF THE INVENTION

The invention relates to absorbent articles, such as diapers and the like. The invention further relates to a package comprising an array of compressed absorbent articles which are housed in a flexible outer casing, such as a film.

### BACKGROUND OF THE INVENTION

Absorbent articles such as diapers and the like are frequently stacked into an array which is compressed before packaging. These sorts of absorbent articles are generally non-uniform in their cross-section resuling in uneven distrubution of the compression force.

EP 0 780 325, published on June 25th 1997, discloses a package comprising an array of compressed articles. The compression of the array is made more uniform by redistributing the orientation of the articles before compression.

Nevertheless the additional steps required to redistribute the orientation of the articles may be difficult to achieve at the high manufacturing and packaging speeds at which modern converting lines operate, and may become a rate-limiting step.

The present invention seeks to provide an array of compressed, absorbent articles that makes efficient use of the available packaging volume by folding the absorbent articles before compression packaging The compressed absorbent articles can be compressed to a smaller volume without causing damage or a significant reduction in the performance of the absorbent articles. Smaller volumes may enable less packaging material, for example less film packaging material to be required and/or the free space inside shipping units may be reduced.

### SUMMARY OF INVENTION

The absorbent articles comprise topsheet, backsheet, and an absorbent core positioned between the topsheet and the backsheet. The absorbent article further comprises fastening elements. The absorbent article has a longitudinal axis and comprises a front waist region, a central absorbent region, and a back waist region. The front waist region and the back waist region comprise at least one of, or both of, the topsheet and the backsheet. The central absorbent region comprises topsheet, backsheet and absorbent core.

The absorbent articles are folded by a first fold extending transversely across the central absorbent region so that the front waist region and the back waist region are positioned in side-by-side configuration. Preferably the topsheet of the front waist region is brought into contact with the topsheet of the back waist region so that the inside of the absorbent article (wearer-side) is substantially within the folded article.

The absorbent articles are further folded by a second fold extending transversely across the front and back waist regions, wherein the second fold extends transversely across the front and back waist regions along a line which lies between the outward edge of the absorbent core and the inner edge of the fastening elements.

### BRIEF DESCRIPTION OF THE DRAWINGS

Figure 1 is a plan view of an absorbent article shown in the flat state.
Figures 2 and 3 are schematic views of a folded absorbent article ("bi-folded").
Figure 4 is a schematic view of a folded absorbent article suitable for use in the array of the present invention.
Figure 5 is a schematic view of an array of absorbent articles according to the present invention.

### DETAILED DESCRIPTION OF THE INVENTION

"Absorbent Articles" as defined herein may include diapers, adult incontinence products, feminine hygiene products, etc. Preferably, absorbent hygiene articles are disposable. "Disposable" refers to devices which are generally not intended to be laundered or otherwise restored or reused (i.e., they are intended to be discarded after a single use and, may be recycled, composted, or otherwise disposed of).

According to the present invention, the absorbent articles further comprise fastening elements which are used to securely close the absorbent article around the waist of the wearer when the absorbent article is donned. Typically such absorbent articles are referred to as "taped diapers" because the fastening elements comprise tapes, such as adhesive tapes, which are affixed to landing zones, and which may be repeatedly unfastened and refastened as needed. The fastening elements may comprise "hook-and-loop" elements.

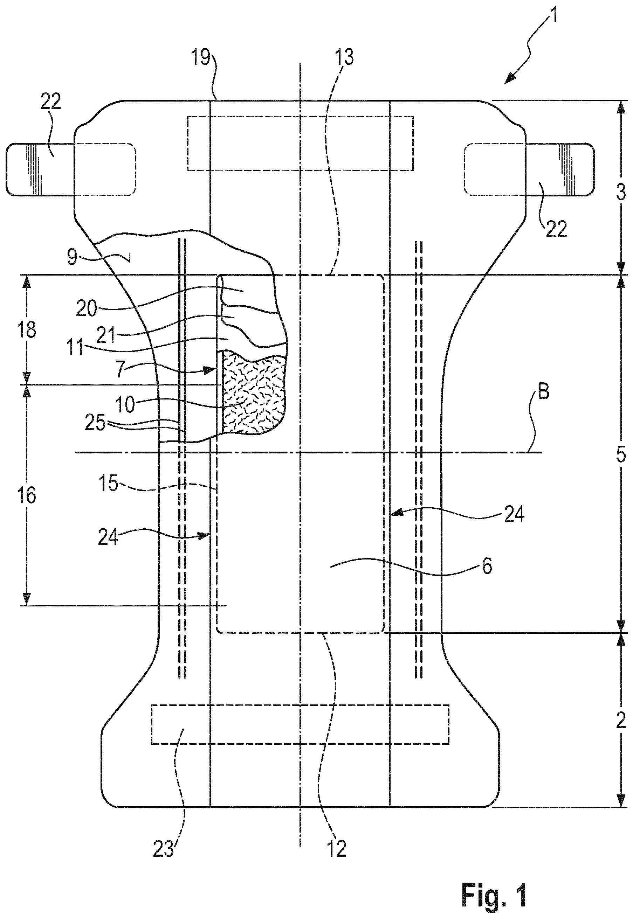

Figure 1 shows an exemplary absorbent article comprising a liquid pervious topsheet 6 that faces the wearer's body, a liquid impervious backsheet 9 that faces the wearer's clothing and an absorbent core 7 interposed between the liquid pervious topsheet 6 and the backsheet 9. The absorbent article comprises a front waist region 2, a central absorbent region 5, and a back waist region 3.

The absorbent core comprises absorbent material 10, typically cellulose fibres, absorbent polymer materials (also known as absorbent gelling material or AGM), or mixtures thereof. The periphery of the absorbent core 7 is defined by the outer absorbent core edges wherein the absorbent core front waist edge 12 and back waist edges 13 run between the longitudinal edges 15 generally parallel to the transverse axis A of the absorbent hygiene article. The longitudinal dimension of the absorbent core 7 extends along the longitudinal axis A from the absorbent core front waist edge 12 to the absorbent core back waist edge 13. The absorbent core 7 also has a transverse dimension extending along the transverse axis B, which is running perpendicular to the longitudinal axis A. The absorbent core 7 comprises an absorbent core crotch region 16 and an absorbent core back waist region 18 which extends from the absorbent core crotch region 16 to the absorbent core back waist edge 13.

The absorbent hygiene article may further comprise an acquisition system disposed between the topsheet 6 and a wearer facing side of the absorbent core 7, for instance between the cover layer 11 of the absorbent core 7 and the topsheet 6. The acquisition system may be in direct contact with the absorbent core 7. The acquisition system may comprise a single layer or multiple layers, such as an upper acquisition layer 20 facing towards the wearer's skin and a lower acquisition layer 21 facing the garment of the wearer.

In case the layer(s) 20, 21 of the acquisition system extend beyond the absorbent material 10, then the limits of the absorbent core are considered, for the purposes of the present invention, to be the outermost edges of the acquisition system or of the absorbent material 10, whichever extends furthest outwards.

As illustrated in Figure 1 the absorbent article 1 is designed to be held in place about the wearer by attaching the fastening elements 22 on back waist region 3 to at least a portion of the front waist region 2 to form leg opening(s) and an article waist. The disposable absorbent hygiene article is provided with a re-closable fastening system joined to the chassis for securing the absorbent hygiene article about a wearer. The fastening system may include at least one fastening elements 22 and at least one landing zone 23. Equally, in an alternative embodiment, the fastening elements 22 may be provided on the front waist region and the landing zone may be provided on the back waist region.

The absorbent article 1 may further comprise elastic members such as leg cuffs. The term "leg cuffs" as used herein comprises leg gasketing systems, leg elastics 25 and barrier cuffs. Suitable leg cuffs are described for example U.S. 3,860,003; U.S. 4,808,178; U.S. 4,695,278 and U.S. 4,795,454.

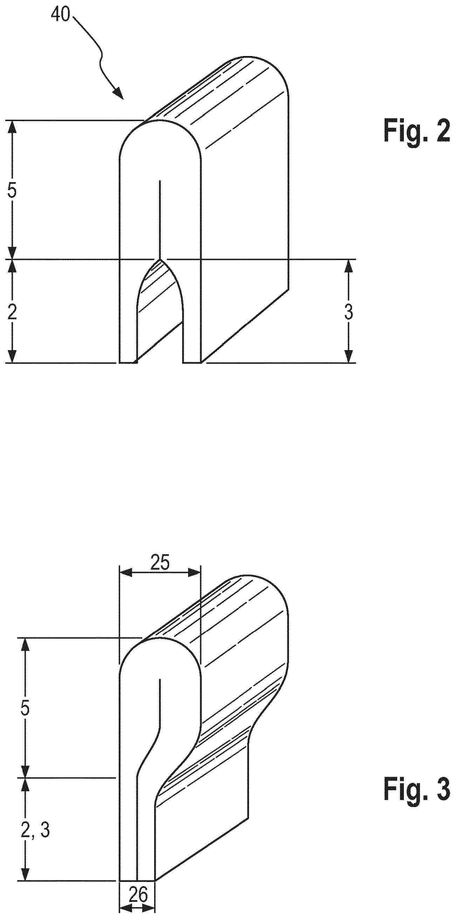

Figure 2 shows an absorbent article, for example the absorbent article of Figure 1, after the article has been folded by a first fold 40 extending transversely across the central absorbent region 5' so that the front waist region 2 and the back waist region 3 are positioned in side-by-side configuration. Figure 3 shows the absorbent article in which the topsheet in the front waist region 2 is brought into contact with the topsheet of the back waist region 3. The first fold 40 extends through the absorbent core 7 which has a generally thicker caliper 25 than the combined caliper of the front and back waist regions 26, The configurations shown in Figures 2 and 3 are referred to herein as a "bi-folded diaper". Typically, a bi-folded diaper is a diaper folded once on itself at its crotch region; such bi-folded diapers have a rounded upper section characterized by a high compression resistance, which corresponds to the crotch region of the unfolded diaper, and a lower section with a low compression resistance, which corresponds to the waist regions of the unfolded diaper.

The first fold 40 may fold the absorbent article along a transverse line with an equal length along the longitudinal axis A of the absorbent article on either side of the first fold 40. Accordingly the leading edge and the trailing edge of the absorbent article are adjacent in the bi-folded diaper. Alternatively the first fold 40 may fold the absorbent article into unequal lengths along the longitudinal axis A of the absorbent article.

The first fold 40 may fold the absorbent core along a transverse line with an equal length along the longitudinal axis A of the absorbent core on either side of the first fold 40. Accordingly the front edge 12 and the back edge 13 are adjacent in the bi-folded diaper. Alternatively the first fold 40 may fold the absorbent core into unequal lengths along the longitudinal axis A of the absorbent core.

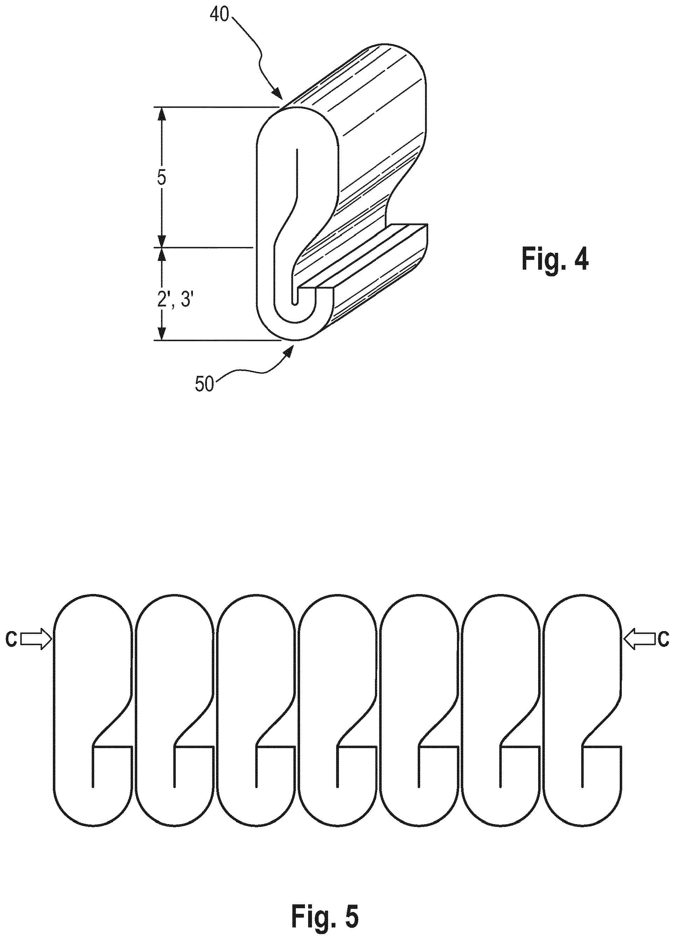

Figure 4 shows the absorbent article after the article has been folded by a second fold 50 extending transversely across the waist region 2', 3'. The second fold 50 is configured to lie outboard of the edges 12, 13 of the absorbent core 7, but inboard of the fastening member 22 to avoid any damage to the fastening member 22 which preferably should remain flat, unfolded, throughout.

Subsequent to the folding operations the absorbent articles are preferably assembled in an array as shown in Figure 5. The array of absorbent article may then be subject to compression prior to inserting the array of absorbent articles into a package, such as a flexible sleeve which forms an outer casing. Compression should be applied in the sense indicated by the arrows C in Figure 5.

The terms "compression" or "compressibility" as used herein are intended to mean the reduction in volume when a predetermined force is applied to an absorbent article or to an array of absorbent articles. This reduction in volume may be between 20% and 70% of the uncompressed volume.

Preferably the present invention may provide absorbent articles which are more compact for the user to handle, and/or which may be more intuitive for the user upon opening up the folded article because it is more obvious which is the back and which is the front of the article; and/or which maybe more hygienic because the inside of the diaper is protected from the outside environment by the double-folded configuration. Preferably the present invention may further provide packages which are stable with regards to shape (tending towards a rectangular design for a better fit when the package is displayed e.g. on supermarket shelves) and has an improved appearance.

The folding process may be carried out by any method known in the art. For example US 8,986,184, "Apparatuses and Methods for Folding an Absorbent Article", issued on March 24^{th} 2015, incorporated herein by reference, discloses folding processes.

The packaging process may be carried out by any method known in the art. For example EP 0 780 325, "Package Comprising an Array of Compressed Absorbent Articles, published on June 25^{th} 1997, incorporated herein by reference, discloses packaging processes, in particular as shown in Figures 6 and 7 of EP 0 780 325. Moreover, in the present invention, the orientation of adjacent articles may be varied, for example by "head to tail" orientation as shown in Figure 9 of EP 0 780 325.

The topsheet 6 may be a nonwoven material made of synthetic fibers alone or in combination with natural fibers. Examples of natural fibers may include cotton, cellulosic natural fibers, such as fibers from hardwood sources, softwood sources, starches or other non-wood plants. The synthetic fibers can be any material, such as, but not limited to, those selected from the group consisting of polyolefins (polypropylene and polypropylene copolymers, polyethylene and polyethylene copolymers), polyesters (e.g., polyethylene terephthalate), polyethers, polyamides, polyesteramides, polyvinylalcohols, polyhydroxyalkanoates, polysaccharides, and combinations thereof. Further, the synthetic fibers can be a single component (i.e. a single synthetic material or a mixture makes up the entire fiber), bi-component (i.e. the fiber is divided into regions, the regions including two or more different synthetic materials or mixtures thereof and may include coextruded fibers and core and sheath fibers) and combinations thereof. Bi-component fibers can be used as a component fiber of the nonwoven material, and/or they may be present to act as a binder for the other fibers present in the nonwoven material. Any or all of the fibers may be treated before, during, or after manufacture to change any desired properties of the fibers. Preferably, the topsheet comprises at least 20% of synthetic fibers, or at least 30% of synthetic fibers or at least 50% of synthetic fibers. In some embodiments, the topsheet comprises 100% of synthetic fibers. Synthetic fibers are preferably thermoplastic fibers. Preferably, the topsheet is made of a nonwoven material made of a polyolefin, such as polyethylene, polypropylene or mixtures thereof. The topsheet may be a multilayer nonwoven web, i.e. a laminate. The laminate may comprise spunbond layer(s) (S), and/or meltblown layer(s) (M), and/or carded layer(s) (C). Suitable laminates include, but are not limited to, SS, SSS, SMS or SMMS. In some embodiments, the topsheet is a spunbond nonwoven material, such as a mono layer spunbond (S), or a dual layer spunbond (SS) or a nonwoven material comprising more than two layers, such as a spunbond nonwoven with three layers (SSS).

The backsheet 9 may be vapor pervious but liquid impervious. The backsheet may prevent the fluids absorbed and contained in the absorbent structure from wetting materials that contact the absorbent article such as underpants, pants, pyjamas, undergarments, and shirts or jackets, thereby acting as a barrier to fluid transport. The backsheet can also allow the transfer of at least water vapor, or both water vapor and air through it. The backsheet may be elastic or inelastic. In some embodiments the backsheet may be a nonwoven fabric. In some embodiments, the backsheet may comprise a laminate of a nonwoven and a thin plastic film such as a thermoplastic film having a thickness of 0.012 mm to 0.051 mm. Suitable backsheet films include those manufactured by Tredegar Industries Inc. of Terre Haute, Ind. and sold under the trade names X15306, X10962, and X10964; or those manufactured by Clopay.

The absorbent core 7 typically comprises absorbent material 10 such as cellulose fibers, modified cellulose fibers (cellulose fibers and modified cellulose fibers are typically referred to in the art as "air-felt"), particulate absorbent polymer material, absorbent foams, tissue, or mixtures thereof. "Particulate absorbent polymer material" as used herein refers to substantially water-insoluble polymer particles that can absorb at least 5 times their weight of a 0.9% saline solution in deionized water as measured using the Centrifuge Retention Capacity test (Edana 441.2-01). Suitable particulate absorbent polymer material can be selected among polyacrylates and polyacrylate based materials, such as for example partially neutralized cross-linked polyacrylates or acid polyacrylate. Examples of polyacrylate based polymers very slightly cross-linked, or substantially not crosslinked at all are described in WO 07/047598.

In some embodiments, the absorbent core 7 may comprise less than 5% by weight of cellulose, more preferably less than 2% and most preferably is cellulose free. The resulting absorbent structures have a reduced thickness in the dry state compared to conventional absorbent structure comprising cellulosic fibers. The reduced thickness helps to improve the fit and comfort of the absorbent article for the wearer.

In some embodiments, the absorbent core 7 may comprise one or more absorbent structures, each absorbent structure comprising a nonwoven substrate layer supporting particulate absorbent polymer material, said particulate absorbent polymer material being immobilized on the nonwoven substrate layer by a thermoplastic adhesive composition, which preferably forms a fibrous network over the particulate absorbent polymer material. Suitable thermoplastic adhesive compositions includes hot melt adhesives comprising at least a thermoplastic polymer in combination with a plasticizer and other thermoplastic diluents such as tackifying resins and additives such as antioxidants. Exemplary suitable hot melt adhesive materials are described in EP 1447067 A2. In some embodiments, the thermoplastic polymer has a molecular weight of more than 10,000 and a glass transition temperature usually below room temperature or comprised from -6 °C to 16°C. In some embodiments, the concentration of the thermoplastic polymer in a hot melt is in the range of about 20 to about 40% by weight. The thermoplastic polymers may be water insensitive. Exemplary polymers are (styrenic) block copolymers including A-B-A triblock structures, A-B diblock structures and (A-B)n radial block copolymer structures wherein the A blocks are non-elastomeric polymer blocks, typically comprising polystyrene, and the B blocks are unsaturated conjugated diene or (partly) hydrogenated versions of such. The B block is typically isoprene, butadiene, ethylene/butylene (hydrogenated butadiene), ethylene/propylene (hydrogenated isoprene), and mixtures thereof. The thermoplastic adhesive composition is generally present in the form of fibres forming a fibrous network over the particulate absorbent polymer material. The fibres may have an average thickness from about 1 µm to about 100 µm, or from about 25 µm to about 75 µm, and an average length from about 5 mm to about 50 cm. The thermoplastic adhesive composition may be applied at an amount of from 0.5 to 30 g/m², or from 1 to 15 g/m², or from 1 and 10 g/m² or even from 1.5 and 5 g/m² per substrate layer. In some embodiments, the particulate absorbent polymer material is distributed in clusters of particles on the nonwoven substrate layer. In some embodiments, the absorbent structure comprises less than 5%, or less than 2% by weight of cellulose, or is cellulose free. The nonwoven substrate layer may enclose the absorbent polymer material and the thermoplastic composition or a separate nonwoven substrate layer may cover the absorbent polymer material and the thermoplastic composition. These nonwoven substrate layers are therefore often referred to as core wrap or core cover. The core wrap or core cover may consist of a first, upper nonwoven web 11 towards the body-facing surface of the absorbent article and of a second, lower nonwoven web (not shown in the figure) towards the garment-facing surface of the absorbent article. The first and second nonwoven webs may be continuously or intermittently bonded to each other around their perimeters. The first and second substrate layers may be made of the same nonwoven webs or may be made of different nonwoven webs, i.e. the first, upper substrate layer may be fluid pervious whereas the second, lower substrate layer may be fluid impervious. In a multilayer absorbent core, one or more layers of a substrate (e.g. a nonwoven web) may additionally be placed within the absorbent core to at least partially separate and segment the particulate absorbent polymer material. The core wrap or core cover may be present in any types of absorbent core.

The dimensions and values disclosed herein are not to be understood as being strictly limited to the exact numerical values recited. Instead, unless otherwise specified, each such dimension is intended to mean both the recited value and a functionally equivalent range surrounding that 30 value. For example, a dimension disclosed as "40 mm" is intended to mean "about 40 mm".

## Claims

1. An absorbent article comprising topsheet (6), backsheet (9), and an absorbent core (7) positioned between the topsheet (6) and the backsheet (9), wherein the absorbent article comprises:
a front waist region (2) and a back waist region (3), wherein the front waist region (2) and the back waist region (3) comprise at least one of, or both of, the topsheet (6) and the backsheet (9); and a central absorbent region (5), wherein the central absorbent region (5) comprises topsheet (6), backsheet (9) and absorbent core (7); the absorbent article further comprises fastening elements (22); and the absorbent article comprises a longitudinal axis, (A),
wherein the absorbent articles comprise a first fold (40) extending transversely across the central absorbent region (5) so that the front waist region (2) and the back waist (3) region are positioned in side-by-side configuration; and the absorbent article further comprises a second fold (50) extending transversely across the front and back waist regions (2, 3), and
wherein the second fold (50) extends transversely across the front and back waist regions (2, 3) along a line which lies between the outward edge of the absorbent core and the inner edge of the fastening elements (22).

2. The absorbent article according to Claim 1 wherein the topsheet (6) of the front waist region (2) is brought into contact with the topsheet (6) of the back waist region (3) so that the inside of the absorbent article is within the folded article.

3. The absorbent article according to either Claim 1 or 2 wherein the fastening elements (22) comprise fastening tapes.

4. The absorbent article according to Claim 3 wherein the fastening tapes comprise adhesive tapes, or hook-and-loop fastenings, or combinations thereof.

5. The absorbent article of any of the previous claims wherein the first fold (40) extends transversely across the central absorbent region (5) and wherein the central absorbent region (5) is bisected into two halves of equal length in the direction of the longitudinal axis (A) by the line of the first fold (40).

6. The absorbent article according to any of claims 1 to 4 wherein the first fold (40) extends transversely across the central absorbent region (5) and wherein the central absorbent region (5) is divided into two parts of unequal length in the direction of the longitudinal axis (A) by the line of the first fold (40).

7. An array of absorbent articles comprising a plurality of the absorbent articles according to any of claims 1 to 6.

8. An array of absorbent articles comprising a plurality of the absorbent articles according to any of claims 1 to 6, wherein the absorbent articles are compressed.

9. A package of absorbent articles comprising an array of a plurality of the absorbent articles according to claim 8 and a flexible outer casing, wherein the flexible outer casing at least partially, and preferably completely, surrounds the array of compressed absorbent articles.

10. The package of claim 9 wherein the flexible outer casing comprises a flexible film.

## Patentansprüche

1. Absorptionsartikel, umfassend eine obere Lage (6), eine untere Lage (9) und einen Absorptionskern (7), der zwischen der oberen Lage (6) und der unteren Lage (9) angeordnet ist, wobei der Absorptionsartikel umfasst:
einen vorderen Taillenbereich (2) und einen hinteren Taillenbereich (3), wobei der vordere Taillenbereich (2) und der hintere Taillenbereich (3) mindestens eine von, oder beide, der oberen Lage (6) und der unteren Lage (9) umfassen; und einen mittleren Absorptionsbereich (5), wobei der mittlere Absorptionsbereich (5) obere Lage (6), untere Lage (9) und Absorptionskern (7) umfasst; wobei der Absorptionsartikel ferner Befestigungselemente (22) umfasst; und der Absorptionsartikel eine Längsachse (A) umfasst,
wobei die Absorptionsartikel eine erste Falte (40) umfassen, die sich quer über den mittleren Absorptionsbereich (5) erstreckt, so dass der vordere Taillenbereich (2) und der hintere Taillenbereich (3) in einer Seite-an-Seite-Konfiguration positioniert sind; und der Absorptionsartikel ferner eine zweite Falte (50) umfasst, die sich quer über den vorderen und hinteren Taillenbereich (2, 3) erstreckt, und wobei sich die zweite Falte (50) quer über den vorderen und hinteren Taillenbereich (2, 3) entlang einer Linie erstreckt, die zwischen dem äußeren Rand des Absorptionskerns und dem inneren Rand der Befestigungselemente (22) liegt.

2. Absorptionsartikel nach Anspruch 1, wobei die obere Lage (6) des vorderen Taillenbereichs (2) in Kontakt mit der oberen Lage (6) des hinteren Taillenbereichs (3) gebracht wird, so dass sich die Innenseite des Absorptionsartikels innerhalb des gefalteten Artikels befindet.

3. Absorptionsartikel nach Anspruch 1 oder 2, wobei die Befestigungselemente (22) Befestigungsbänder umfassen.

4. Absorptionsartikel nach Anspruch 3, wobei die Befestigungsbänder Klebebänder oder Klettverschlüsse oder Kombinationen davon umfassen.

5. Absorptionsartikel nach einem der vorhergehenden Ansprüche, wobei sich die erste Falte (40) quer über den mittleren Absorptionsbereich (5) erstreckt und wobei der mittlere Absorptionsbereich (5) in der Richtung der Längsachse (A) durch die Linie der ersten Falte (40) in zwei Hälften gleicher Länge halbiert wird.

6. Absorptionsartikel nach einem der Ansprüche 1 bis 4, wobei sich die erste Falte (40) quer über den mittleren Absorptionsbereich (5) erstreckt und wobei der mittlere Absorptionsbereich (5) in Richtung der Längsachse (A) durch die Linie der ersten Falte (40) in zwei Teile ungleicher Länge unterteilt wird.

7. Anordnung von Absorptionsartikeln, umfassend eine Vielzahl der Absorptionsartikel nach einem der Ansprüche 1 bis 6.

8. Anordnung von Absorptionsartikeln, umfassend eine Vielzahl der Absorptionsartikel nach einem der Ansprüche 1 bis 6, wobei die Absorptionsartikel komprimiert sind.

9. Verpackung von Absorptionsartikeln, umfassend eine Anordnung einer Vielzahl der Absorptionsartikel nach Anspruch 8 und eine flexible Außenhülle, wobei die flexible Außenhülle die Anordnung von komprimierten Absorptionsartikeln zumindest teilweise und vorzugsweise vollständig umgibt.

10. Verpackung nach Anspruch 9, wobei die flexible Außenhülle eine flexible Folie umfasst.

## Revendications

1. Article absorbant comprenant une feuille de dessus (6), une feuille de fond (9), et une âme absorbante (7) positionnée entre la feuille de dessus (6) et la feuille de fond (9), dans lequel l'article absorbant comprend :
une région de ceinture avant (2) et une région de ceinture arrière (3), dans lequel la région de ceinture avant (2) et la région de ceinture arrière (3) comprennent au moins l'une, ou l'une et l'autre, de la feuille de dessus (6) et de la feuille de fond (9) ; et une région absorbante centrale (5), dans lequel la région absorbante centrale (5) comprend une feuille de dessus (6), une feuille de fond (9) et une âme absorbante (7) ; l'article absorbant comprend en outre des éléments de fixation (22) ; et l'article absorbant comprend un axe longitudinal, (A),
dans lequel les articles absorbants comprennent un premier pli (40) s'étendant transversalement à travers la région absorbante centrale (5) de sorte que la région de ceinture avant (2) et la région de ceinture arrière (3) sont positionnées dans une configuration côte à côte ; et l'article absorbant comprend en outre un deuxième pli (50) s'étendant transversalement à travers les régions de ceinture avant et arrière (2, 3), et dans lequel le deuxième pli (50) s'étend transversalement à travers les régions de ceinture avant et arrière (2, 3) le long d'une ligne qui se situe entre le bord extérieur de l'âme absorbante et le bord intérieur des éléments de fixation (22).

2. Article absorbant selon la revendication 1 dans lequel la feuille de dessus (6) de la région de ceinture avant (2) est mise en contact avec la feuille de dessus (6) de la région de ceinture arrière (3) de sorte que l'intérieur de l'article absorbant se trouve à l'intérieur de l'article plié.

3. Article absorbant selon la revendication 1 ou 2 dans lequel les éléments de fixation (22) comprennent des bandes de fixation.

4. Article absorbant selon la revendication 3 dans lequel les bandes de fixation comprennent des bandes adhésives, ou des fixations à crochets et boucles, ou des combinaisons de celles-ci.

5. Article absorbant selon l'une quelconque des revendications précédentes dans lequel le premier pli (40) s'étend transversalement à travers la région absorbante centrale (5) et dans lequel la région absorbante centrale (5) est coupée en deux moitiés de longueur égale dans la direction de l'axe longitudinal (A) par la ligne du premier pli (40).

6. Article absorbant selon l'une quelconque des revendications 1 à 4 dans lequel le premier pli (40) s'étend transversalement à travers la région absorbante centrale (5) et dans lequel la région absorbante centrale (5) est divisée en deux parties de longueur inégale dans la direction de l'axe longitudinal (A) par la ligne du premier pli (40).

7. Ensemble d'articles absorbants comprenant une pluralité des articles absorbants selon l'une quelconque des revendications 1 à 6.

8. Ensemble d'articles absorbants comprenant une pluralité des articles absorbants selon l'une quelconque des revendications 1 à 6, dans lequel les articles absorbants sont comprimés.

9. Conditionnement d'articles absorbants comprenant un ensemble d'une pluralité des articles absorbants selon la revendication 8 et une enveloppe externe souple, dans lequel l'enveloppe externe souple entoure au moins partiellement, et de préférence complètement, l'ensemble d'articles absorbants comprimés.

10. Conditionnement selon la revendication 9 dans lequel l'enveloppe externe souple comprend un film souple.
